# EUROPÄISCHE PATENTANMELDUNG

(11) **EP 3 567 598 A1**
(43) Veröffentlichungstag der Anmeldung: **13.11.2019**
(21) Anmeldenummer: 18171646.5
(22) Anmeldetag: 09.05.2018
(51) Int. Cl.: G16H 30/40

(54) **PRÜFUNG EINES INTERVENTIONELLEN SUPERPOSITIONSBILDES**

(71) Anmelder: Siemens Healthcare GmbH, 91052 Erlangen (DE); Friedrich-Alexander-Universität Erlangen-Nürnberg, 91054 Erlangen (DE)
(72) Erfinder: Breininger, Katharina, 91052 Erlangen (DE); Kowarschik, Markus, 90408 Nürnberg (DE); Pfister, Marcus, 91088 Bubenreuth (DE)

(57) **Zusammenfassung**

Die Erfindung betrifft ein Verfahren zur automatischen Prüfung eines Superpositionsbildes (SUP, MODSUP, KORSUP) einer interessierenden Körperregion eines Untersuchungsobjektes (6). Das Verfahren umfasst folgende Schritte:
- Ermitteln (S11, S21, S31) wenigstens einer Referenzposition eines Objektes (AKREF, U, CL, ABZW) in einem Referenzbild (REF),
- Ermitteln (S12, S221, S222, S32) einer aktuellen Position des Objektes (AKDU) in einem aktuellen Durchleuchtungsbild (DU, MODU),
- Erzeugen (S13, S231, S232, S33) des Superpositionsbildes durch Überlagern des aktuellen Durchleuchtungsbildes und des Referenzbildes,
- Ermitteln (S14, S241, S242, S341, S342) wenigstens eines Parameters (PAR ABW, PAR KORABW, PAR MODABW) kennzeichnend ein Abweichungsmaß (ABW, KORABW, MODABW) zwischen Referenzposition und aktuelle Position des Objektes in dem Superpositionsbild,
- Anzeigen (S15, S251, S252, S351, S352) des Abweichungsmaßes für einen Benutzer.

## Beschreibung

Die Erfindung betrifft neuartige Mittel zur Prüfung eines interventionellen Superpositionsbildes und insbesondere ein Verfahren zur automatischen Beurteilung einer Superpositionsbildqualität in Echtzeit.

Fluoroskopie-gesteuerte Interventionen werden typischerweise auf Angiographie-Anlagen bzw. mobilen C-Bogen-Systemen durchgeführt, die jeweils die Röntgen-Bildgebung verwenden. Die Fluoroskopie wird dann eingesetzt, wenn eine "Echtzeit-Untersuchung" eines Patienten erforderlich ist. Typische Einsatzmöglichkeiten der Fluoroskopie sind die Funktionsprüfung eines orthopädischen Gelenkersatzes, die Kontrolle von Kathetern und Herzschrittmachern, die Verteilung eines Kontrastmittels im Magen-Darmtrakt, die Bewegung verschiedener Körperteile und -organe (z. B. Schluckvorgang und Bewegung der Speiseröhre) oder die minimal-invasive Reparatur von Aneurysmen, bspw. in der abdominellen Aorta.

Ein abdominelles Aortenaneurysma (AAA) ist eine Gefäßaussackung. Behandelt wird dies bspw. durch Einsetzen eines Stent Grafts. Über beide Leisten werden dazu Führungsdrähte und Katheter in die Aorta eingebracht, über die ein oder mehrere Stent Grafts, also Gefäßplastiken oder Endoprothesen, eingebracht und platziert werden. Bei komplexen Interventionen werden nicht nur die Aorta, sondern auch die verschiedenen Abzweigungen, wie bspw. Nierenarterien mit speziellen Stents versorgt. Der endgültige Stent wird also aus mehreren verschiedenen einzeln einzubringenden 'Teilstents' zusammengesetzt. Ziel beim Einsetzen dieser Stent Grafts ist eine präzise Platzierung, ohne dabei wichtige Gefäßabgänge zu überdecken. Zum Darstellen der Gefäße werden während des gesamten Eingriffs kontinuierlich zwei-dimensionale Fluoroskopie-Aufnahmen mittels einer C-Bogen-Röntgenanlage gemacht, um den Vorschub, die Position und/oder die Lage von Katheter, Führungsdrähten, Endoprothesen und/oder Blutgefäßen zu überwachen und zu kontrollieren. Auch können auf C-Bogen-Systemen Angiographien (typischerweise zwei-dimensionale Projektionen unter Kontrastmittelgabe) angefertigt werden, die Informationen über einen Blutgefäßverlauf geben. Vorteilhaft können diese Angiographien den aktuellen Fluoroskopie-Aufnahmen als sogenannte 'Roadmaps' überlagert werden. Um jedoch die Gabe von nierenschädigendem Kontrastmittel zu minimieren, können einer aktuellen Fluoroskopie-Aufnahme Informationen aus Referenzbildern (üblicherweise prä-operative, drei-dimensionale Computertomographie- oder MRT-Aufnahmen) anatomisch korrekt überlagert werden. Die Referenzbilder zeigen typischerweise zusätzliche anatomische Details oder Referenzpunkte, wie bspw. Gefäßabzweigungen oder in der Fluoroskopie nicht aufgelöste Tiefeninformation und dienen als Positionierhilfe für das einzubringende medizinische Material. Dreidimensionale Datensätze können zudem grundsätzlich jeder Fluoroskopie-Aufnahme überlagert werden, unabhängig von der am C-Bogen eingestellten Angulation, also unabhängig von der für die Bildgebung eingenommene Blickrichtung auf die interessierende Körperregion des Patienten.

Zweckmässig zur Erstellung einer Überlagerung von Fluoroskopie-Aufnahmen und Referenzbilddaten ist eine Segmentierung von anatomischen Objektes und/oder weiteren Strukturen mittels bekannter Segmentierungsverfahren. Voraussetzung zur Überlagerung ist eine Registrierung der prä-operativen Referenzbilder zum C-Arm mittels bekannter Registrierungsverfahren, um die Übereinstimmung zwischen Referenz- und aktuellem Bild zu erreichen. Daneben sind Verfahren bekannt, um in den Fluoroskopie-Aufnahmen medizinische Instrumente bzw. Material zu erkennen und zu verfolgen, wie bspw. in LESSARD, S. et. al.: "Automatic detection of selective arterial devices for advanced visualization during abdominal aortic aneurysm endovascular repair", in: Med Eng Phys., 2015, Vol. 37(10), pp. 979-986 für den genannten Anwendungsfall eines Aortenaneurysmas beschrieben.

Besonders bei komplexen Prozeduren leidet jedoch die Genauigkeit bzw. die Qualität der Überlagerung, also die Übereinstimmung zwischen zwei-dimensionaler Fluoroskopie-Aufnahme und den Referenzbilddaten. Grund hierfür kann zum einen eine Patientenbewegung sein, da insbesondere minimal-invasive Eingriffe häufig mit lediglich lokaler Anästhesie ausgeführt werden. Zum anderen sorgen regelmäßig auch die eingebrachten Operationsinstrumente für eine Verformung der Anatomie. Je größer die Abweichungen zwischen aktueller Aufnahme und Referenzdaten sind, umso weniger ist die Anzeige der Überlagerung für den Betrachter von Nutzen. Dieser muss während des Eingriffs die Abweichung visuell erkennen und 'gedanklich' korrigieren. Alternativ dazu kann die Überlagerung auch manuell oder benutzerinitiiert automatisch korrigiert, bzw. durch individuelle Vorgaben des Betrachters semi-automatisch korrigiert werden, wie bsw. in der deutschen Patentanmeldung DE 102010012621 A1 oder in TOTH et al., "Adaption of 3D Models to 2D X-Ray Images during Endovascular Abdominal Aneurysm Repair", In: Navab N., Hornegger J., Wells W., Frangi A. (eds) Medical Image Computing and Computer-Assisted Intervention -- MICCAI 2015. Lecture Notes in Computer Science, vol 9349, Springer, Cham, beschrieben.

Diese Vorgehensweise erfordert zumindest ein Erkennen der Abweichung durch den Benutzer, unterbricht die Arbeit von Operationspersonal und ist darüber hinaus zeitaufwändig und insbesondere für ungeübte Betrachter schwierig und damit fehleranfällig.

Demgegenüber ist es Aufgabe der vorliegenden Erfindung, alternative Mittel bereit zu stellen, die es erlauben, automatisch, zuverlässig und schnell die Qualität eines Superpositionsbildes im Hinblick auf anatomische Abweichungen zu erfassen. Insbesondere ist es Aufgabe der vorliegenden Erfindung, die Qualität des Superpositionsbildes in Echtzeit bzw. Quai-Echtzeit zu erfassen.

Diese Aufgabe wird gelöst durch ein Verfahren zur automatischen Prüfung eines Superpositionsbildes einer interessierenden Körperregion eines Untersuchungsobjektes, entsprechende Recheneinheit und medizinische Bildgebungsanlage, entsprechendes Computerprogramm und entsprechenden computerlesbaren Datenträger gemäß den unabhängigen Ansprüchen. Bevorzugte und/oder alternative, vorteilhafte Ausgestaltungsvarianten sind Gegenstand der abhängigen Ansprüche.

Nachstehend wird die erfindungsgemäße Lösung der Aufgabe in Bezug auf das beanspruchte Verfahren als auch in Bezug auf die beanspruchten Vorrichtungen beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können gegenständliche Ansprüche (die beispielsweise auf ein Verfahren gerichtet sind) auch mit Merkmalen, die in Zusammenhang mit einer der Vorrichtungen beschrieben oder beansprucht sind, weitergebildet sein. Die entsprechenden funktionalen Merkmale des Verfahrens werden dabei durch entsprechende gegenständliche Module oder Einheiten ausgebildet.

Die vorliegende Erfindung betrifft in einem ersten Aspekt ein Verfahren zur automatischen Prüfung eines Superpositionsbildes einer interessierenden Körperregion eines Untersuchungsobjektes.

Im Folgenden wird ohne Beschränkung der Allgemeinheit von einem Patienten als Untersuchungsobjekt ausgegangen, wobei es sich meist um einen Menschen handelt. Grundsätzlich kann der Patient auch ein Tier sein. Im Folgenden werden daher die beiden Begriffe "Untersuchungsobjekt" und "Patient" synonym verwendet. Das Untersuchungsobjekt kann alternativ eine Pflanze oder ein nicht-lebender Gegenstand, z.B. ein historisches Artefakt oder dergleichen sein. Die interessierende Körperregion beschreibt einen Bereich, Teilbereich und/oder Körperteil des Patienten, der gewisse anatomische Strukturen, Organe und/oder Gewebe umfasst, die mittels medizinischer Bildgebungsanlage abgebildet werden sollen. Bspw. kann die interessierende Körperregion das Abdomen, der Kopf und/oder der Thorax sein. Das Superpositionsbild darstellend die interessierende Körperregion kann bspw. den Verlauf von Blutgefäßen innerhalb der interessierenden Körperregion darstellen. Alternativ kann das Superpositionsbild auch knöcherne Strukturen wie Schädel, Hüfte oder Wirbelsäule darstellen. Das Superpositionsbild ist jedoch darauf nicht beschränkt, es kann im Wesentlichen alle anatomischen Objekte oder Strukturen darstellen, die sich mittels Referenzbild und/oder Durchleuchtungsbild (in aller Regel ohne Verwendung von Kontrastmittel erzeugt) abbilden lassen.

Das Verfahren umfasst eine Vielzahl von Schritten. In einem ersten Schritt erfolgt ein Ermitteln einer Referenzposition wenigstens eines Objektes in einem Referenzbild. In einem zweiten Schritt erfolgt ein Ermitteln einer aktuellen Position des Objektes in einem aktuellen Durchleuchtungsbild. Das aktuelle Durchleuchtungsbild entspricht erfindungsgemäß einer aktuellen Fluoroskopie-Aufnahme, welches zu einem Zeitpunkt im Verlauf einer Intervention, also eines medizinischen Eingriffs erfasst wird. Das aktuelle Durchleuchtungsbild ist bevorzugt eine zweidimensionale Projektion, die mittels einer C-Bogen-Röntgen-Anlage erfasst wird, sie kann jedoch auch einem dreidimensionalen Datensatz entsprechen. Das Durchleuchtungsbild wird typischerweise ohne Kontrastmittelgabe erfasst, kann aber auch unter Kontrastmittelgabe erzeugt worden sein. Das Referenzbild entspricht erfindungsgemäß einem bevorzugt dreidimensionalen Bilddatensatz. Dieser wurde zu einem Referenzzeitpunkt, bevorzugt vor dem medizinischen Eingriff erfasst bzw. erzeugt. Das Referenzbild ist bspw. als Computer-Tomographie-Aufnahme oder Magnetresonanztomographie-Aufnahme ausgebildet. Das Referenzbild kann auch als zweidimensionales Bild ausgestaltet sein. Aktuellem Durchleuchtungsbild und Referenzbild ist gemein, dass beide dieselbe interessierende Körperregion des Patienten abbilden und somit dieselben anatomischen Objekte bzw. Strukturen innerhalb der interessierenden Körperregion mittelbar oder unmittelbar umfassen. Insbesondere können die Bilder jeweils Blutgefäße, also wenigstens ein und dasselbe Blutgefäß, abbilden. Bevorzugt sind mehrere Blutgefäße bzw. ein Gefäß und Gefäßabgänge, Verästelungen und/oder ein ganzer Gefäßbaum von der abgebildeten, interessierenden Körperregion umfasst. Das Ermitteln einer Referenzposition wenigstens eines Objektes in dem Referenzbild erfolgt für wenigstens ein abgebildetes Objekt manuell und/oder mittels an sich bekannter Segmentierungsverfahren. Das wenigstens eine Objekt kann insbesondere ein Blutgefäß sein, es kann aber auch eine bestimmte Knochen- oder Gewebestruktur, ein bestimmtes Organ oder dergleichen sein. Für den Fall, dass das Objekt ein Blutgefäß ist, kann das Ermitteln der Position in Referenzbild und/oder aktuellem Durchleuchtungsbild ein Ermitteln eines Verlaufs des Blutgefäßes umfassen. Die Segmentierung kann insbesondere ein Ermitteln von Außenkonturen der Blutgefäße, von approximierten Umfängen, von Centerlines der Blutgefäße, von bestimmten Landmarken wie bspw. Gefäßabgänge und/oder Abzweigungen, von Durchmessern der Blutgefäße oder dergleichen umfassen.

In einer bevorzugten Ausführungsform des erfindungsgemäßen Verfahrens, in der das Objekt ein Blutgefäß ist, umfasst das Ermitteln der aktuellen Position des Objektes in dem aktuellen Durchleuchtungsbild ein Erkennen bzw. Segmentieren eines in dem Blutgefäß befindlichen Instruments, einer Endoprothese wie bspw. eines Stents oder Stentteils, einer Aortenklappe oder Herzklappe, der Außenkontur und/oder des Volumens des Blutgefäßes. Dabei geht die Erfindung davon aus, dass die Position bzw. Lage medizinischer Instrumente, soweit sie innerhalb des Blutgefäßes liegen, im Wesentlichen die aktuelle Position bzw. den aktuellen Verlauf des Blutgefäßes repräsentieren.

In einem weiteren Schritt erfolgt ein Erzeugen des Superpositionsbildes durch Überlagern des aktuellen Durchleuchtungsbildes und des Referenzbildes. Üblicherweise wird dazu das Referenzbild, meistens zu Beginn des medizinischen Eingriffs, zu bestehenden Durchleuchtungsbildern registriert, d.h. anhand verschiedener gemeinsamer Merkmale (z.B. Gefäßverläufe oder Knochenstrukturen) in Übereinstimmung gebracht. Bevorzugt ist die Übereinstimmung so gut, dass sie auch für andere Blickrichtungen valide ist. Es können an sich bekannte Verfahren zur Registrierung zum Einsatz kommen.

In einem weiteren Schritt erfolgt ein Ermitteln wenigstens eines Parameters kennzeichnend ein Abweichungsmaß zwischen Referenzposition und aktueller Position Objektes in dem Superpositionsbild. Bei der Überlagerung von Referenzbild und aktuellem Durchleuchtungsbild können sich bspw. aufgrund von Patientenbewegung und/oder für den bevorzugten Fall Objekt = Blutgefäß wegen der in das Blutgefäß eingebrachten medizinischen Instrumente Unterschiede zwischen aktuellem Verlauf und Referenzverlauf ergeben. Die aktuelle Position kann ggü. der Referenzposition verschoben, verdreht, gedehnt, gestaucht und/oder dergleichen sein. Diese Abweichungen werden durch die Superposition sichtbar und in dem aktuellen Schritt automatisch quantisiert. Der ermittelte Parameter gibt insofern die 'Güte' bzw. Qualität der Überlagerung im Superpositionsbild an. Er kann bspw. für den Fall Objekt = Blutgefäß einen Oberflächenanteil eines Stents in dem aktuellen Durchleuchtungsbild angeben, der in dem Superpositionsbild außerhalb des Blutgefäßes gemäß Referenzverlauf liegt. Der Parameter kann einen Abstand zwischen Gefäß-Außenkonturen in Referenzbild und aktuellem Durchleuchtungsbild angeben. Der Parameter kann auch angeben, ob ein medizinisches Instrument repräsentierend die aktuelle Position bzw. den aktuellen Verlauf eines Blutgefäßes innerhalb oder außerhalb des Blutgefäßes gemäß Referenzposition bzw. -verlauf liegt. Eine Vielzahl von anderen Parametern ist hier denkbar, um die Abweichungen zu quantifizieren. Erfindungsgemäß wird wenigstens ein Parameter, bevorzugt jedoch eine Vielzahl von verschiedenen Parametern berechnet.

In einem weiteren Schritt erfolgt ein Anzeigen des Abweichungsmaßes für einen Benutzer. Das Superpositionsbild wird einem Chirurgen bzw. Operationspersonal typischerweise im Rahmen eines medizinischen Eingriffs als visuelle Unterstützung zur Anzeige gebracht, bspw. auf einem Monitor einer C-Bogen-Röntgen-Anlage. Die Erfinder haben nun erkannt, dass es besonders vorteilhaft für das Operationspersonal ist, wenn er neben dem Superpositionsbild an sich auch das berechnete Maß der Abweichung zwischen aktuellem Durchleuchtungsbild und Referenzbild angezeigt bekommt. Derart kann er die Qualität der Superposition berücksichtigen. Besonders bevorzugt wird das Abweichungsmaß zusammen mit bzw. in der Superposition visuell veranschaulicht. Andere Anzeige- bzw. Ausgabemodi sind ebenfalls denkbar. Eine visuelle Anzeige kann bspw. erfolgen, indem der ermittelte Wert für wenigstens einen Parameter angezeigt wird. Alternativ kann in Abhängigkeit wenigstens eines Parameters eine seinem ermittelten Wert entsprechende, farbige Signallampe eingefügt werden, bspw. 'grün' für keine oder nur geringe Abweichungen, und 'rot' für größere bzw. kritische Abweichungen. Um das Abweichungsmaß weiter zu veranschaulichen, können in dem Superpositionsbild segmentierte Strukturen gemäß Referenzposition und aktueller Position von Objektes farbig markiert werden, bspw. Gefäß- und/oder Stent-Außenkonturen.

In einer vorteilhaften Ausführungsform des erfindungsgemäßen Verfahrens werden die Schritte
- Ermitteln einer aktuellen Position des Objektes in dem aktuellen Durchleuchtungsbild,
- Erzeugen des Superpositionsbildes durch Überlagern des aktuellen Durchleuchtungsbildes und des Referenzbildes,
- Ermitteln wenigstens eines Parameters kennzeichnend ein Abweichungsmaß zwischen Referenzposition und aktueller Position des Objektes in dem Superpositionsbild, und
- Anzeigen des Abweichungsmaßes für einen Benutzer
in Quasi-Echtzeit ausgeführt werden. Derart ergeben sich im Verlauf des medizinischen Eingriffs keine störenden bzw. für den Patienten gesundheitlich kritischen Verzögerung. Quasi-Echtzeit beschreibt dabei eine maximale Zeitdauer, die bis zur Anzeige der Superposition zusammen mit dem Abweichungsmaß verstreicht. Diese ist erfindungsgemäß kleiner als die Zeit zwischen der Erfassung zweier Durchleuchtungsbilder im Verlauf des medizinischen Eingriffs.

In einer besonders bevorzugten Ausführungsform des erfindungsgemäßen Verfahrens erfolgt die Segmentierung insbesondere des aktuellen Durchleuchtungsbildes unter Anwendung eines neuronalen Netzes oder mittels einer Template-Matching Technik. Der zeitlimitierende Schritt bei dem erfindungsgemäßen Verfahren ist die Erkennung, also die Segmentierung der abgebildeten Strukturen oder Objekte, insbesondere der Blutgefäße. Die Erfinder haben nun erkannt, dass sich der Prozess durch Anwendung der genannten Techniken bis hin zu Quasi-Echtzeit beschleunigen lässt. Die Wahl einer geeigneten Segmentierungsmethode ist abhängig von individuellen Anwendungsfall bzw. der interessierenden Körperregion.

Das so genannte Template Matching ist ein klassisches Aufgabengebiet der Objekterkennung in Bildern und nutzt Informationen über Struktur, Form, Orientierung und/oder Farbe der Objekte. Die Beschriebenen Eigenschaften werden durch ein Muster vorgegeben und ein Bild nach diesem Muster abgesucht. Erfüllt ein identifiziertes Objekt in dem Bild ein vorbestimmtes Übereinstimmungsmaß, wird es als das gesuchte Objekt selektiert.

Ein neuronales Netz, insbesondere ein künstliches neuronales Netz orientiert sich am Aufbau eines biologischen neuronalen Netzes wie bspw. einem menschlichen Gehirn. Ein künstliches neuronales Netz umfasst zwischen einer Eingabe- und einer Ausgabeschicht bevorzugt eine Vielzahl von weiteren Schichten jeweils umfassend wenigstens einen Knoten. Jeder Knoten entspricht dabei einer Verarbeitungseinheit, analog einem biologischen Neuron. Knoten innerhalb einer Schicht des Netzes können über gerichtete Verbindungen (Kanten) mit Knoten anderer Schichten verbunden sein. Die Verbindungen definieren den Datenfluss innerhalb des Netzes. Jeder Knoten repräsentiert folglich eine Operation, die auf die Eingabedaten angewendet wird. Ferner verfügt jeder Knoten bzw. jede seiner Verbindungen über einen Gewichtungsparameter. Über diesen Gewichtungsparameter wird der Einfluss bzw. die Wichtigkeit der Ausgabe eines Knotens als Eingabewert für einen Empfängerknoten definiert. In der Trainingsphase, die bevorzugt als überwachtes Lernen ausgeführt wird, 'lernt' das Künstliche Neuronale Netz anhand von Trainingsdaten die Gewichtungsparameter für alle Knoten bzw. Verbindungen und passt diese solange an, bis die Ausgabeschicht des Netzes die korrekten Ausgabewerte liefert. Typsicherweise wird ein erster Teil von Netzschichten zur Merkmalsextraktion in Bildern verwendet. Die identifizierten Merkmale werden dann als Eingabewerte für einen zweiten Teil von Netzschichten, den sog. Klassifikatoren, verwendet, welche den extrahierten Merkmalen in den Bildern vorhandene Objekte zuordnet.

In einer weiteren Ausführungsform des erfindungsgemäßen Verfahrens werden die Schritte
- Ermitteln einer aktuellen Position des Objektes in dem aktuellen Durchleuchtungsbild,
- Erzeugen des Superpositionsbildes durch Überlagern des aktuellen Durchleuchtungsbildes und des Referenzbildes,
- Ermitteln wenigstens eines Parameters kennzeichnend ein Abweichungsmaß zwischen Referenzposition und aktueller Position des Objektes in dem Superpositionsbild, und
- Anzeigen für einen Benutzer des Abweichungsmaßes.
kontinuierlich für eine Vielzahl aufeinander folgender Durchleuchtungsbilder durchgeführt. Diese Vorgehensweise beruht auf der Erkenntnis, dass sich durch Patientenbewegung und/oder das kontinuierliche Verschieben, Verlagern oder Einbringen weiterer medizinsicher Instrumente oder Materialien in ein Blutgefäß während eines medizinischen Eingriffs die aktuelle Position des wenigstens einen Objektes kontinuierlich ggü. der Referenzposition verändert wird. Durch die kontinuierlich wiederholte Ausführung des erfindungsgemäßen Verfahrens wird eine permanente Kontrollmöglichkeit geschaffen. In diesem Sinne eignet sich die vorliegende Erfindung für eine Vielzahl von medizinischen Eingriffen, wie bspw. den Einsatz von Stents (intrakranial, Abdomen, Thorax, Herzkranzgefäße) oder Herz- bzw. Aortenklappen oder Roboter-gestützte Interventionen.

In einer weiteren Ausführungsform des erfindungsgemäßen Verfahrens berücksichtigt das Ermitteln des Parameters kennzeichnend das Abweichungsmaß Vorwissen über segmentierte Strukturen aus wenigstens einem zuvor analysierten Durchleuchtungsbild. Diese Vorgehensweise nutzt die Erkenntnis, dass für ein vorheriges, nicht notwendigerweise das direkt vorhergehende, Durchleuchtungsbild bereits Strukturen erkannt und Parameter kennzeichnend das Abweichungsmaß ermittelt bzw. berechnet wurden. Diese können im Sinne einer Plausibilitätsprüfung bei der Auswertung des aktuellen Durchleuchtungsbildes bzw. des dazu gehörigen Superpositionsbildes herangezogen werden, bspw. indem automatisch geprüft wird, ob ein zuvor und aktuell ermittelter Parameter kennzeichnend das Abweichungsmaß innerhalb (Ergebnis ist plausibel) oder außerhalb (Ergebnis ist nicht plausibel) eines vorbestimmten Wertebereichs liegen. Hierbei wird davon ausgegangen, dass Veränderungen der aktuellen Position, insbesondere dem aktuellen Verlauf eines Blutgefäßes, zwischen benachbarten oder zeitlich eng bei einander liegenden Durchleuchtungsbildern gering sind.

In einer anderen Ausführungsform des erfindungsgemäßen Verfahrens sind folgende Schritte umfasst:
- Automatisches Erzeugen eines korrigierten Superpositionsbildes basierend auf dem Parameter kennzeichnend das Abweichungsmaß zwischen Referenzposition und aktueller Position des Objektes
- Ermitteln wenigstens eines Parameters kennzeichnend ein korrigiertes Abweichungsmaß zwischen Referenzposition und aktueller Position des Objektes in dem korrigierten Superpositionsbild und
- Anzeigen des korrigierten Abweichungsmaßes für einen Benutzer.
Liegt der ermittelte, wenigstens eine Parameter kennzeichnend das Abweichungsmaß oberhalb einer vorbestimmten Toleranz bzw. einem vorbestimmten Schwellwert, ist das ein Anzeichen dafür, dass die Überlagerung zwischen aktuellem Durchleuchtungsbild und Referenzbild nicht ausreichend gut ist und korrigiert werden sollte. Für die Erzeugung eines korrigierten Superpositionsbildes können die eingangs genannten Lehren aus der deutschen Patentanmeldung DE 102010012621 A1 oder TOTH et al., "Adaption of 3D Models to 2D X-Ray Images during Endovascular Abdominal Aneurysm Repair", in: Navab N., Hornegger J., Wells W., Frangi A. (eds) Medical Image Computing and Computer-Assisted Intervention -- MICCAI 2015. Lecture Notes in Computer Science, vol 9349, Springer, Cham, heran gezogen werden. Durch die Korrektur der Überlagerung werden die Strukturen in dem korrigierten Superpositionsbild besser überlagert. Im Wesentlichen basiert die Korrektur auf einer Anpassung der Referenzposition eines Objektes und insbesondere des Referenzverlaufs eines Blutgefäßes an die aktuelle Position bzw. den aktuellen Verlauf des aktuellen Durchleuchtungsbilds. Das korrigierte Superpositionsbild weist eine erhöhte klinische Aussagekraft für Operationspersonal auf. Die Korrektur kann, bspw. in Abhängigkeit des ermittelten Parameters kennzeichnend das Abweichungsmaß automatisch durchgeführt oder dem Benutzer automatisch zur Bestätigung vorgeschlagen werden. Alternativ kann das Operationspersonal die Korrektur manuell triggern. Das korrigierte Superpositionsbild wird dann im weiteren Verlauf des erfindungsgemäßen Verfahrens ausgewertet und ein Parameter für ein korrigiertes Abweichungsmaß ermittelt. Die beschriebene Vorgehensweise kann iterativ ausgebildet sein und solange durchgeführt werden, bis der wenigstens eine Parameter kennzeichnend ein Abweichungsmaß einen vorbestimmten Schwellwert unterschreitet. Insbesondere kann die hier beschriebene Vorgehensweise mit der folgenden Ausführungsform kombiniert und auf ein modifiziertes Superpositionsbild angewandt werden.

In einer weiteren bevorzugten Ausführungsform des erfindungsgemäßen Verfahrens sind auch folgende Schritte umfasst:
- Basierend auf dem Parameter kennzeichnend das Abweichungsmaß Ableiten wenigstens eines Aufnahmeparameters für eine medizinische Bildgebungsanlage, wenn der Parameter für das Abweichungsmaß einen vorbestimmten Schwellwert überschreitet
- Erfassen eines modifizierten Durchleuchtungsbildes unter Anwendung des Aufnahmeparameters
- Ermitteln einer aktuellen Position des Objektes in dem modifizierten Durchleuchtungsbild
- Erzeugen eines modifizierten Superpositionsbildes durch Überlagern des modifizierten Durchleuchtungsbildes und des Referenzbildes
- Ermitteln wenigstens eines Parameters kennzeichnend ein modifiziertes Abweichungsmaß zwischen Referenzposition und aktueller Position des Objektes in dem Superpositionsbild
- Anzeigen des modifizierten Abweichungsmaßes für einen Benutzer.
Weist wenigstens einer der ermittelten Parameter kennzeichnend ein Abweichungsmaß einen Wert auf, der außerhalb eines vorbestimmten Wertebereichs oder oberhalb eines vorbestimmten Schwellwerts liegt, ist das ein Anzeichen dafür, dass die Abweichungen der Lage, Position bzw. Verlauf der segmentierten Strukturen in aktuellem Durchleuchtungsbild und Referenzbild so groß sind, dass eine sinnvolle Überlagerung in dem Sinne nicht erreicht werden kann, dass die Überlagerung nicht wie oben beschrieben korrigiert werden kann. In dieser Situation schlägt die Erfindung vor, in Abhängigkeit des Parameters kennzeichnend das Abweichungsmaß wenigstens einen Aufnahmeparameter für das Erfassen eines neuen, modifizierten Durchleuchtungsbildes abzuleiten. Insofern kann das erfindungsgemäße Verfahren wenigstens einen Aufnahmeparameter, bspw. eine geänderte Angulation, eine geänderte Kollimation, eine geänderte Röntgenröhrenspannung, oder dergleichen vorschlagen und ein modifiziertes Durchleuchtungsbild erfassen, welches sich besser für eine wie oben beschriebene Korrektur eignet. Die Ableitung eines Aufnahmeparameters kann Benutzer-getriggert sein oder automatisch erfolgen. Die Erfassung des modifizierten Durchleuchtungsbildes mit dem abgeleiteten Aufnahmeparameter kann Benutzer-getriggert erfolgen. Das erfindungsgemäße Verfahren wird dann im Anschluss anhand des modifizierten Durchleuchtungsbildes durchgeführt und ein Parameter kennzeichnend ein modifiziertes Abweichungsmaß ermittelt. Liegt der Parameter kennzeichnend das Abweichungsmaß nach Ausführung der beschriebenen Schritte noch nicht innerhalb der Toleranz, kann das Verfahren wiederholt durchgeführt, erneut ein Aufnahmeparameter abgeleitet und ein modifiziertes Durchleuchtungsbild erfasst werden usw.

In dieser Ausführungsform wird also ein gravierender ,Mismatch' zwischen Referenzbild und aktuellem Durchleuchtungsbild erkannt. Dies kann durch ein zu kleines Bildfeld, fehlende strukturelle Information über die interessierende Körperregion und/oder eine ungünstigen Angulation/Kollimierung des aktuellen Durchleuchtungsbildes bedingt sein. Infolgedessen wird wenigstens ein Aufnahmeparameter zur Aufnahme eines weiteren Durchleuchtungsbildes vorgeschlagen, das voraussichtlich eine Korrektur erlaubt.

In einem zweiten Aspekt betrifft die vorliegende Erfindung eine Recheneinheit zur automatischen Prüfung eines Superpositionsbildes einer interessierenden Körperregion eines Untersuchungsobjektes. Die Recheneinheit weist Mittel zum Durchführen des erfindungsgemäßen Verfahrens auf.

In einer bevorzugten Ausführungsform ist die Recheneinheit mit einer medizinischen Bildgebungsanlage derart verbunden, dass Steuersignale in Bezug auf korrigierte Aufnahmeparameter bzw. aktuelle und/oder modifizierte Durchleuchtungsbilder übertragen werden können. Weiter vorteilhaft ist die Recheneinheit mit einer Anzeigeeinheit einer medizinischen Bildgebungsanlage zur Anzeige eines (korrigierten/modifizierten) Superpositionsbildes und/oder eines (korrigierten/modifizierten) Abweichungsmaßes verbunden.

Vorteilhaft ist die Recheneinheit in die medizinische Bildgebungsanlage integriert. Alternativ kann die Recheneinheit auch entfernt bzw. abgelegen davon angeordnet sein. Die Recheneinheit kann ausgebildet sein, insbesondere den Schritt des Ermittelns wenigstens eines Parameters kennzeichnend ein Abweichungsmaß zwischen Referenzposition und aktueller Position des Objektes in einem Superpositionsbild, aber auch das gesamte erfindungsgemäße Verfahren, für eine medizinische Bildgebungsanlage oder für eine Vielzahl von Anlagen durchzuführen, z.B. in einem Radiologie-Zentrum oder Krankenhaus umfassend mehrere medizinische Bildgebungsanlagen.

In einem dritten Aspekt betrifft die Erfindung eine medizinische Bildgebungsanlage in Form eines C-Bogen-Röntgengerätes. Die medizinische Bildgebungsanlage umfasst vorteilhaft eine erfindungsgemäße Recheneinheit. Eine medizinische Bildgebungsanlage ist eine Bildgebungsanlage zum Einsatz in der Medizin. Eine erfindungsgemäße medizinische Bildgebungsanlage verwendet zur Bilderzeugung Röntgenstrahlung. Die medizinische Bildgebungsanlage eignet sich bevorzugt zum Einsatz bei medizinischen Eingriffen, also zur Echtzeit-Bildgebung. Die medizinische Bildgebungsanlage kann insbesondere als mobile C-Bogen-Röntgen-Anlage ausgebildet sein.

Ein vierter Aspekt der vorliegenden Erfindung betrifft ein Computerprogramm mit Programmcode, um das erfindungsgemäße Verfahren zur automatischen Prüfung eines Superpositionsbildes einer interessierenden Körperregion eines Untersuchungsobjektes durchzuführen, wenn das Computerprogramm auf einem Computer, bspw. der erfindungsgemäßen Recheneinheit, ausgeführt wird.

Ein fünfter Aspekt der vorliegenden Erfindung betrifft einen computerlesbaren Datenträger mit Programmcode eines Computerprogramms, um das erfindungsgemäße Verfahren zur automatischen Prüfung eines Superpositionsbildes einer interessierenden Körperregion eines Untersuchungsobjektes durchzuführen, wenn das Computerprogramm auf einem Computer, bspw. der erfindungsgemäßen Recheneinheit, ausgeführt wird. Vorteilhaft kann insbesondere der Schritt des Ermittelns wenigstens eines Parameters kennzeichnend ein Abweichungsmaß zwischen Referenzposition und aktueller Position des Objektes in einem Superpositionsbild auf einem Computer, beispielsweise in einer Recheneinheit einer medizinischen Bildgebungsanlage, ausgeführt werden.

Die Ausführung der Erfindung in Form eines Computerprogramms bzw. eines computerlesbaren Datenträgers umfassend Programmcode eines erfindungsgemäßen Computerprogramms bietet den Vorteil, dass bestehende Computersysteme bzw. Recheneinheiten leicht durch ein Software-Update angepasst werden können, um eine erfindungsgemäße Funktion zu erzielen.

Das Computerprogramm kann alternativ in Form eines Computerprogrammprodukts ausgebildet sein und zusätzliche Einheiten aufweisen. Diese können als Hardware ausgebildet sein, bspw. als Speichermedium, auf welchem das Computerprogramm gespeichert ist, und/oder ein Hardware Schlüssel, um das Computerprogramm nutzen zu können. Alternativ oder zusätzlich können sie als Software ausgebildet sein, bspw. als eine Programm-Dokumentation oder ein Software Schlüssel, um das Computerprogramm nutzen zu können.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Durch diese Beschreibung erfolgt keine Beschränkung der Erfindung auf diese Ausführungsbeispiele. In verschiedenen Figuren sind gleiche Komponenten mit identischen Bezugszeichen versehen. Die Figuren sind in der Regel nicht maßstäblich. Es zeigen:
- FIG 1: eine schematische Darstellung eines erfindungsgemäßen Verfahrens gemäß einem Ausführungsbeispiel der vorliegenden Erfindung,
- FIG 2: eine schematische Darstellung eines erfindungsgemäßen Verfahrens gemäß einem anderen Ausführungsbeispiel der vorliegenden Erfindung,
- FIG 3: eine schematische Darstellung eines erfindungsgemäßen Verfahrens gemäß einem weiteren Ausführungsbeispiel der vorliegenden Erfindung,
- FIG 4: eine Ansicht einer medizinischen Bildgebungsanlage in Form eines C-Bogen-Röntgen-Gerätes gemäß einer Ausführungsform der vorliegenden Erfindung umfassend eine erfindungsgemäßen Recheneinheit gemäß einem Ausführungsbeispiel der vorliegenden Erfindung,
- FIG 5: ein Superpositionsbild gemäß einem Ausführungsbeispiel der vorliegenden Erfindung, und
- FIG 6: ein Superpositionsbild gemäß einem Ausführungsbeispiel der vorliegenden Erfindung.

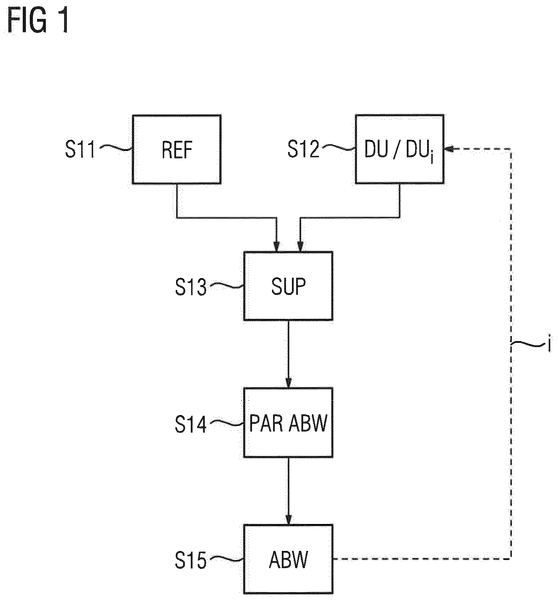

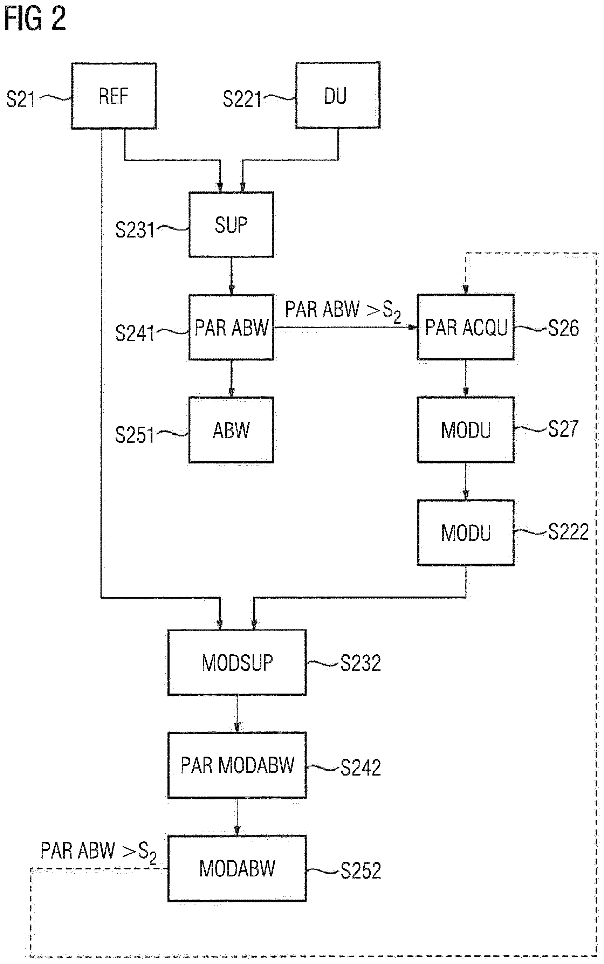

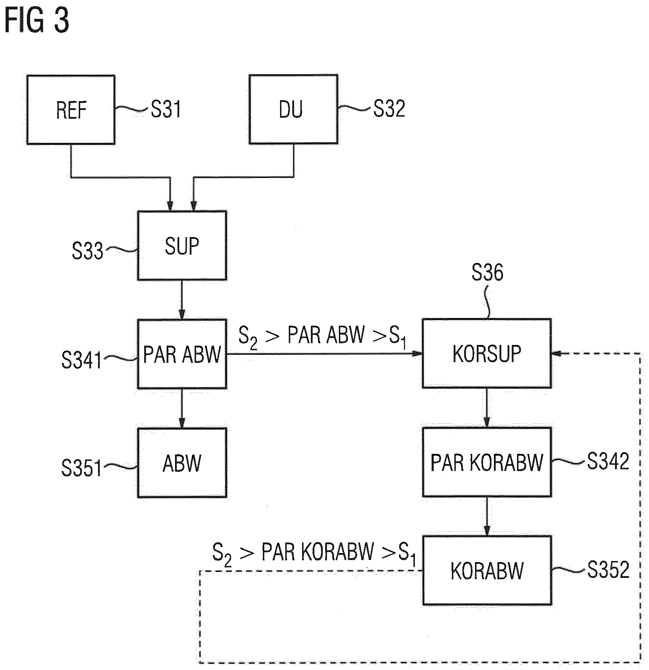

**Figur 1** zeigt eine schematische Darstellung eines erfindungsgemäßen Verfahrens mit einer Vielzahl von Schritten gemäß einem Ausführungsbeispiel der vorliegenden Erfindung. In einem ersten Schritt S11 erfolgt eine Ermittlung einer Referenzposition eines Objektes in Form eines Referenzverlaufs wenigstens eines Blutgefäßes in einem Referenzbild REF. Die Ermittlung erfolgt hier mittels einer Segmentierung, kann aber auch auf andere Art bestimmt werden. Mit anderen Worten wird das Referenzbild nach anatomischen Strukturen 'abgesucht'. Konkret können Außenkonturen oder Außenflächen eines Blutgefäßes, Gefäßklappen, Gefäßabzweigungen, Gefäßvolumen, Gefäßdurchmesser, Gefäß-Centerlines, markante Abstände zu anderen abgebildeten Strukturen oder Organen, diese Organe oder Strukturen selbst oder dergleichen mittels Segmentierungsverfahren erfasst werden. Wenigstens ein abgebildetes Blutgefäß, bspw. die abdominale Aorta wird auf diese Weise analysiert. Das Referenzbild kann ein dreidimensionaler CT-, MR- oder C-Bogen-Bilddatensatz (bspw. eine Rotationsangiographie) oder ein zweidimensionaler Bilddatensatz (bspw. eine mittels C-Bogen-Röntgen-Gerät erfasste Digitale Subtraktionsangiographie). Schritt S11 wird bevorzugt zeitlich losgelöst von den anderen Schritten S12 bis S15 ausgeführt. Ist das Referenzbild bspw. bereits vorverarbeitet, kann vorteilhaft Rechenzeit und Rechenkapazität im Verlauf eines medizinischen Eingriffs eingespart werden. In einem zweiten Schritt S12 erfolgt eine Ermittlung einer aktuellen Position des Objektes in Form eines aktuellen Verlaufs des Blutgefäßes anhand eines aktuellen Durchleuchtungsbildes DU. Dieses wird im Verlauf eines medizinischen Eingriffes mittels einer medizinischen Bildgebungsanlage in Form eines C-Bogen-Röntgen-Gerätes als digitale Röntgenprojektion unter einer bestimmten Angulation des C-Bogen-Systems erfasst. Das Durchleuchtungsbild entspricht einer Momentaufnahme der interessierenden, abgebildeten Körperregion des Patienten und zeigt insbesondere auch medizinisches Gerät, Endoprothesen, Instrumente und dergleichen, die sich in der interessierenden Körperregion und insbesondere in dem Blutgefäß befinden. Schritt S12 umfasst eine Segmentierung dieser Objekte im aktuellen Durchleuchtungsbild DU. Der aktuelle Verlauf des Blutgefäßes wird unter der Annahme ermittelt, dass sich die segmentierten Objekte innerhalb des Blutgefäßes befinden, insofern wird der aktuelle Verlauf des Blutgefäßes durch die Position, Lage, Erstreckung, Ausdehnung, oder dergleichen dieser Objekte angenähert. Sofern das aktuelle Durchleuchtungsbild DU unter Kontrastmittelgabe erzeugt wurde (und ein ausreichender Kontrast zwischen Blutgefäßen und umliegendem Gewebe erzielt wird), kann das Ermitteln auch ein Segmentieren der Außenkontur, bzw. der Gefäßfläche des Blutgefäßes umfassen. Beide Schritte S11 und S12 können zudem optional die Segmentierung von weiteren anatomischen Strukturen wie konkrete Landmarken oder dergleichen umfassen, um eine anschließende Überlagerung der Bilder REF und DU zu erleichtern. Für die Segmentierung des Referenzbildes REF, aber besonders für das aktuelle Durchleuchtungsbild DU kommen erfindungsgemäß Methoden des Template-Matching oder ein künstliches, neuronales Netz zum Einsatz. Dadurch kann die Segmentierung stark beschleunigt werden, wodurch sich das Verfahren grundsätzlich für eine Quasi-Echtzeit-Anwendung eignet. In einem weiteren Schritt S13 erfolgt eine Überlagerung bzw. Superposition des Referenzbildes REF und des aktuellen Durchleuchtungsbildes DU. Mit anderen Worten werden in Schritt S13 die segmentierten und zu einander korrespondierenden Strukturen, insbesondere Blutgefäße, zu einander überlagert. Voraussetzung dafür ist, dass das Referenzbild REF in Bezug auf das Koordinatensystem des C-Bogen-Röntgen-Geräts registriert wurde. Ergebnis von Schritt S13 ist ein Superpositionsbild SUP. Wenn weder eine Patientenbewegung stattgefunden hat, noch die in das Blutgefäß eingebrachten medizinischen Instrumente eine Verformung bzw. Verlagerung des Blutgefäßes verursacht haben, stimmen Referenzverlauf und aktueller Verlauf des Blutgefäßes in dem Superpositionsbild SUP im Wesentlichen überein. Die Abweichungen sind vernachlässigbar. Sofern Patientenbewegung und/oder eine Verlagerung bzw. Verformung des Blutgefäßes durch die eingeführten medizinischen Instrumente stattgefunden hat, zeigt das Superpositionsbild SUP diese Abweichungen bzw. Unterschiede in den Gefäßverläufen auf. Um die Abweichungen für bspw. einen Chirurgen, dem das Superpositionsbild SUP angezeigt wird, besser zu verdeutlichen, können bspw. Außenkonturen von Blutgefäßen, die Lage, Orientierung, Zentrum, Form und/oder Größe von Gefäßabzweigungen in einem Blutgefäß, Landungszonen für Stents, Blutgefäßumfänge oder dergleichen eingefügt, markiert und/oder farblich hervor gehoben werden. In einem weiteren Schritt S14 erfolgt ein Ermitteln wenigstens eines Parameters kennzeichnend ein Abweichungsmaß PAR ABW zwischen den Gefäßverläufen. In diesem Schritt wird die Abweichung der Gefäßverläufe quantisiert. Mögliche Parameter kennzeichnend ein Abweichungsmaß PAR ABW können bspw. sein: ein Abstand zwischen einer Außenkontur des Blutgefäßes gemäß dem Referenzverlauf sowie dem aktuellen Verlauf, ein Anteil einer Blutgefäß-Fläche gemäß dem aktuellen Verlauf, der in dem Superpositionsbild SUP außerhalb der Blutgefäß-Fläche gemäß Referenzverlauf liegt, oder dergleichen. Weitere Ausgestaltungen für den Parameter kennzeichnend ein Abweichungsmaß sind ebenfalls denkbar. Alternativ oder zusätzlich kann in Schritt S14 als Parameter PAR ABW auch ermittelt werden, ob die in das Blutgefäß eingebrachten und gemäß aktuellem Durchleuchtungsbild DU segmentierten medizinischen Instrumente innerhalb oder außerhalb der Blutgefäß-Flächen gemäß Referenzbild REF liegen. In einem letzten Schritt S15 dieses Ausführungsbeispiels des erfindungsgemäßen Verfahrens wird das zuvor ermittelte Abweichungsmaß ABW dem Chirurgen, zur Anzeige gebracht. Dazu wird die Gesamtheit aller ermittelten Parameter kennzeichnend das Abweichungsmaß PAR ABW berücksichtigt und in eine intuitive und für das Operationspersonal geeignete Darstellung überführt. Bspw. kann das Abweichungsmaß ABW mittels einer dem Superpositionsbild SUP überlagerten Ampelanzeige ausgegeben werden. Liegt das Abweichungsmaß innerhalb eines ersten Toleranzbereichs bzw. unterhalb eines ersten Schwellwerts S1, wird die Ampel in dem Superpositionsbild SUP auf 'grün' geschaltet und dem Operationspersonal damit leicht verständlich angezeigt, dass die Überlagerung von Referenzbild REF und aktuellem Durchleuchtungsbild DU eine gute Qualität aufweist. Liegt das ermittelte Abweichungsmaß innerhalb eines zweiten Toleranzbereichs bzw. unterhalb eines zweiten Schwellwerts S2, der größer als der erste Toleranzbereich bzw. der erste Schwellwert S1 ist, wird die Ampel in dem Superpositionsbild SUP auf 'gelb' geschaltet und dem Operationspersonal damit leicht verständlich angezeigt, dass die Überlagerung von Referenzbild REF und aktuellem Durchleuchtungsbild DU eine korrigierbare Qualität aufweist. Eine Korrektur des Superpositionsbildes SUP im Rahmen dieser Erfindung wird weiter unten mit Bezug zu Figur 2 näher beschrieben. Liegt das ermittelte Abweichungsmaß auch außerhalb des zweiten Toleranzbereiches bzw. oberhalb des zweiten Schwellwerts S2, wird die Ampel in dem Superpositionsbild SUP auf 'rot' geschaltet und dem Operationspersonal damit leicht verständlich angezeigt, dass die Überlagerung von Referenzbild REF und aktuellem Durchleuchtungsbild DU aufgrund erheblicher Unterschiede der in beiden Bildern umfassten Strukturen eine unbrauchbare Qualität aufweist und ggf. dass ein neues, modifiziertes Durchleuchtungsbild MODU erfasst werden muss. Diese erfindungsgemäße Vorgehensweise wird weiter unten mit Bezug zu Figur 3 näher beschrieben. Alternativ zur Ampelanzeige kann im Superpositionsbild SUP auch eine Legende umfassend eine Auflistung der ermittelten Werte aller Paramater kennzeichnend das Abweichungsmaß PAR ABW angezeigt werden. Alternative Lösungen für die Anzeige des Abweichungsmaßes ABW sind ebenfalls denkbar. Optional kann erfindungsgemäß vorgesehen sein, dass die Schritte S12 bis S15 wiederholt durchgeführt werden, und zwar für eine Vielzahl, bevorzugt alle im Rahmen eines medizinischen Eingriffs kontinuierlich erfassten Durchleuchtungsbilder DUi. Insofern wird in der i-ten Wiederholschleife (gestrichelter Pfeil) das erfindungsgemäße Verfahren auf das i-te Durchleuchtungsbild DUi angewandt. Insbesondere in der Ausführungsvariante, in welcher für alle aufeinanderfolgenden Durchleuchtungsbilder das Abweichungsmaß ermittelt und angezeigt wird, ist es von Vorteil, dasselbe im Rahmen von Schritt S14 mit dem für das vorherige Durchleuchtungsbild DUi-1 zu vergleichen und dadurch auf Plausibilität zu prüfen. Dies geschieht unter der Annahme, dass die Veränderung im Blutgefäßverlauf zwischen zwei aufeinander folgenden Durchleuchtungsbildern DUi-1 und DUi eher gering sind.

**Figur 3** zeigt eine schematische Darstellung eines erfindungsgemäßen Verfahrens gemäß einem weiteren Ausführungsbeispiel der vorliegenden Erfindung. Auch hier ist das betrachtete Objekt als Blutgefäß ausgebildet. Die Schritte S31, S32, S33, S34 und S35 entsprechen im Wesentlichen den Schritten S11 bis S15 gemäß Figur 1. In diesem Ausführungsbeispiel ergibt Schritt S341, dem Ermitteln wenigstens eines Parameters für ein Abweichungsmaß PAR ABW, dass das Abweichungsmaß ABW bzw. wenigstens einer der ermittelten Parameter PAR ABW größer als ein erster Schwellwert S1 (und kleiner als ein zweiter Schwellwert S2) ist. In dieser Ausführung wird gemäß Schritt S36 automatisch basierend auf dem wenigstens einen Parameter kennzeichnend ein Abweichungsmaß PAR ABW wenigstens ein korrigiertes Superpositionsbild KORSUP erzeugt. Mit anderen Worten wird für die durch den ermittelten Wert dieses Parameters PAR ABW beschriebene Abweichung zwischen Referenzbild REF und aktuellem Durchleuchtungsbild DU eine Operation ermittelt, die angewandt wird, um den ermittelten Referenzverlauf des Blutgefäßes an den aktuellen Verlauf des Blutgefäßes anzugleichen. Die Operation kann als Verschiebung, Dehnung, Stauchung, Drehung Verdrillung und/oder dergleichen des Blutgefäßes oder eines Teils bzw. Abschnitts davon sein. Ergebnis von Schritt S36 ist ein korrigiertes Superpositionsbild KORSUP. Die Korrektur des Referenzverlaufs des Blutgefäßes kann alternativ durch Operationspersonal gestartet bzw. überwacht werden. Es kann bspw. vorgesehen sein, dass der Benutzer eine geeignete Operation aus einer Liste möglicher Operationen auswählt bzw. eine gewünschte Operation eingibt. Für das korrigierte Superpositionsbild KORSUP wird in Schritt S342 wenigstens ein Parameter kennzeichnend ein Abweichungsmaß PAR KORABW ermittelt, in Analogie zu Schritt S341. In Schritt S352 wird das ermittelte korrigierte Abweichungsmaß KORABW, bevorzugt zusammen mit dem korrigierten Superpositionsbild KORSUP dem Operationspersonal angezeigt. Ergibt eine erneute Prüfung, dass das korrigierte Abweichungsmaß KORABW bzw. wenigstens ein Parameter kennzeichnend ein modifiziertes Abweichungsmaß PAR KORABW noch immer oberhalb des ersten vorbestimmten Schwellwerts S1 liegt, können im Rahmen einer Widerholschleife der Schritte S36 bis S352 (gestrichelter Pfeil) weitere modifizierte Superpositionsbilder KORSUP erzeugt und auf ihre Überlagerungsqualität hin überprüft werden, bis der erste Schwellwert S1 unterschritten wird. Die Wiederholschleife kann auch Benutzer-seitig abgebrochen werden, sofern die Überlagerungsqualität des angezeigten korrigierten Superpositionsbild KORSUP ausreichend ist.

**Figur 2** zeigt eine schematische Darstellung eines erfindungsgemäßen Verfahrens gemäß einem anderen Ausführungsbeispiel der vorliegenden Erfindung. Auch hier ist das betrachtete Objekt als Blutgefäß ausgebildet. Die Schritte S21, S221, S231, S241 und S251 entsprechen im Wesentlichen den Schritten S11 bis S15 gemäß Figur 1. In diesem Ausführungsbeispiel ergibt Schritt S241, dem Ermitteln wenigstens eines Parameters für ein Abweichungsmaß PAR ABW, dass das Abweichungsmaß ABW bzw. wenigstens einer der ermittelten Parameter PAR ABW größer als der zweite Schwellwert S2 ist. In dieser Ausführung wird gemäß Schritt S26 automatisch wenigstens ein Aufnahmeparameter PAR ACQU für die medizinische Bildgebungsanlage ermittelt. Bei dem Aufnahmeparameter PAR ACQU handelt es sich insbesondere um eine geänderte Angulation, eine geänderte Kollimierung und/oder eine geänderte Röntgenröhrenspannung. Unter Anwendung des geänderten Aufnahmeparameters PAR ACQU kann gemäß Schritt S27 ein modifiziertes Durchleuchtungsbild MODU erfasst werden, welche gegenüber dem aktuellen Durchleuchtungsbild eine geänderte Blickrichtung, ein geändertes Blickfeld bzw. einen geänderten Kontrast aufweist. Die Bilderfassung kann automatisch oder durch Benutzerbestätigung eines Vorschlags zur Neuerfassung. Der ermittelte Aufnahmeparameter PAR ACQU kann auch durch Operationspersonal angepasst werden. Ziel dieser Vorgehensweise ist es, ein Durchleuchtungsbild MODU zur Verfügung zu stellen, welches mit dem Referenzbild REF überlagert besser als das aktuelle Durchleuchtungsbild DU für eine beschriebene Korrektur eignet bzw. eine Korrektur ganz vermieden werden kann. In Analogie zu Schritt S221 bzw. S12 wird in Schritt S222 auch das modifizierte Durchleuchtungsbild MODU einer Segmentierung unterzogen und dann in Schritt S232 zur Erzeugung eines modifizierten Superpositionsbilds MODSUP mit dem Referenzbild REF überlagert. In den Schritten S242 und S252 erfolgt in Analogie zu den Schritten S241 und S251 ein Ermitteln wenigstens eines Parameters kennzeichnend ein modifiziertes Abweichungsmaß PAR MODABW sowie eine Anzeige des ermittelten modifizierten Abweichungsmaßes MODABW für das Operationspersonal, hier nun lediglich in Bezug auf das modifizierte Superpositionsbild MODSUP. Ergibt eine erneute Prüfung, dass das modifizierte Abweichungsmaß MODABW bzw. wenigstens ein Parameter kennzeichnend ein modifiziertes Abweichungsmaß PAR MODABW noch immer oberhalb des zweiten vorbestimmten Schwellwerts S2 liegt, können im Rahmen einer Widerholschleife der Schritte S26 bis S252 (gestrichelter Pfeil) weitere veränderte Aufnahmeparameter PAR ACQU für das Erfassen weiterer modifizierter Durchleuchtungsbilder MODU abgeleitet werden, solange bis der zweite Schwellwert S2 unterschritten wird. Die Wiederholschleife kann auch Benutzer-seitig abgebrochen werden, sofern die Überlagerungsqualität des angezeigten modifizierten Superpositionsbild MODSUP ausreichend ist.

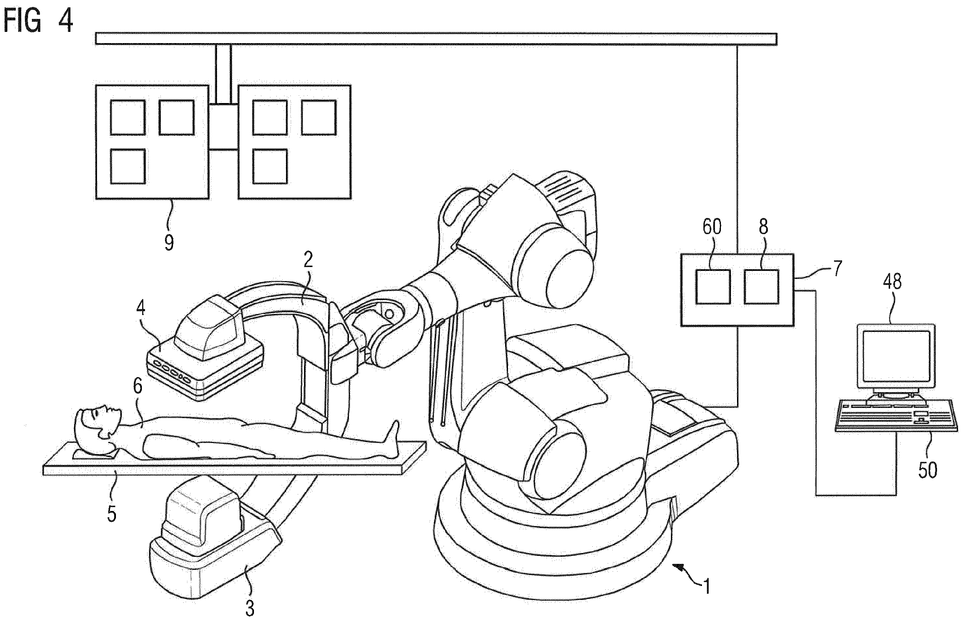

**Figur 4** zeigt eine erfindungsgemäße medizinische Bildgebungsanlage 1 in einem Ausführungsbeispiel. Hier handelt es sich um ein monoplanes angiographisches Röntgensystem 1 in Form einer C-Bogen-Röntgenanlage mit einem von einem Ständer in Form eines sechsachsigen Knickarmroboters gehaltenen C-Bogen 2, an dessen Enden eine Röntgenstrahlungsquelle, beispielsweise ein Röntgenstrahler 3 mit Röntgenröhre und Kollimator, und ein Röntgenbilddetektor 4 angebracht sind. Das erfindungsgemäße angiographische Röntgensystem 1 ist insbesondere um Drehzentren und Drehachsen in der C-Bogen-Ebene des Röntgenbilddetektors 4 drehbar. Anstelle des Ständers kann das angiographische Röntgensystem auch eine normale decken- oder bodenmontierte Halterung für den C-Bogen 2 aufweisen oder mobil ausgebildet sein. Im Strahlengang des Röntgenstrahlers 3 befindet sich auf einer Patientenliege 5 ein zu untersuchender Patient 6 als Untersuchungsobjekt. Die medizinische Bildgebungsanlage 1 umfasst in dieser Ausführung eine Recheneinheit 7 in Form eines Computers. Die Recheneinheit 7 ist grundsätzlich ausgebildet, die mittels des Röntgenbilddetektors 4 erfassten Bildsignale, bspw. aktuelles oder modifiziertes Durchleuchtungsbild DU, MODU zu empfangen und zu verarbeiten. Die verarbeiteten Röntgenbilder, bspw. (korrigiertes/modifiziertes) Superpositionsbild SUP, KORSUP, MODSUP können dann auf Displays einer Monitorampel 9 betrachtet werden. Die Recheneinheit 7 kann alternativ als eigenständige Recheneinheit ausgebildet sein. Die Recheneinheit 7 steht ferner mit einer Ausgabeeinheit 48 und einer Eingabeeinheit 50 in Datenkommunikation. Die Ausgabeeinheit 48 dient beispielsweise zur graphischen Anzeige von Auswahloptionen für abgeleitete Aufnahmeparameter PAR ACQU oder Korrekturoperationen an Operationspersonal. Die Eingabeeinheit 50 dient beispielsweise der Auswahl und oder Bestätigung von abgeleiteten Aufnahmeparameter PAR ACQU oder Korrekturoperationen durch das Operationspersonal. Bei der Ausgabeeinheit 48 kann es sich beispielsweise um einen LCD-, Plasma- oder OLED-Bildschirm handeln. Es kann sich weiterhin um einen berührungsempfindlichen Bildschirm handeln, welcher auch als Eingabeeinheit 50 ausgebildet ist. Bei der Eingabeeinheit 50 handelt es sich beispielsweise um eine Tastatur, eine Maus, einen sogenannten "Touch-Screen" oder auch um ein Mikrofon zur Spracheingabe. Die Eingabeeinheit 50 kann auch eingerichtet sein, um Bewegungen eines Benutzers zu erkennen und in entsprechende Befehle zu übersetzen.

Die Recheneinheit 7 steht auch mit dem Röntgenbilddetektor 4 und dem Röntgenstrahler 3 zum Datenaustausch in Verbindung. Es können zum Beispiel Steuersignale in Bezug auf abgeleitete Aufnahmeparameter PAR ACQU, mit denen ein modifiziertes Durchleuchtungsbild MODU erfasst werden soll. Die Datenverbindungen sind jeweils in bekannter Weise kabelgebunden oder kabellos realisiert.

Die Recheneinheit 7 umfasst eine Bildverarbeitungseinheit 8, die eingerichtet ist, Röntgenbilder, insbesondere Referenzbilder REF und/oder Durchleuchtungsbilder DU, MODU zu analysieren und enthaltene Strukturen wie bspw. Blutgefäße und/oder medizinische Instrumente zu segmentieren. Die Bildverarbeitungseinheit 8 ist ferner eingerichtet, um durch eine geeignete Registrierung von Referenzbildern REF und Durchleuchtungsbildern DU, MODU Superpositionsbilder SUP, KORSUP, MODSUP zu erzeugen. Die Bildverarbeitungseinheit 8 ist ferner ausgebildet, Parameter kennzeichnend ein Abweichungsmaß PAR ABW, PAR KORABW, PAR MODABW aus einem Superpositionsbild SUP, KORSUP, MODSUP zu ermitteln und daraus Korrekturoperationen zur Erzeugung eines korrigierten Superpositionsbildes KORSUP abzuleiten und, falls erforderlich, basierend auf den ermittelten Parametern PAR ABW einen Aufnahmeparameter PAR ACQU abzuleiten. Dazu ist die Bildverarbeitungseinheit 8 auch eingerichtet, einen Parameter kennzeichnend ein Abweichungsmaß PAR ABW mit vorbestimmten Schwellwerten S1, S2 bzw. Toleranzbereichen zu vergleichen. Die Schwellwerte S1, S2 können z.B. in einem von der Recheneinheit 7 ebenfalls umfassten Speicher 60 zum automatischen Abruf hinterlegt sein. Sie können insbesondere vom durchgeführten medizinischen Eingriff bzw. der interessierenden Körperregion abhängen und auch durch Operationspersonal individuell angepasst werden.

Die Recheneinheit 7 kann mit einem computerlesbaren Datenträger zusammenwirken, insbesondere um durch ein Computerprogramm mit Programmcode ein erfindungsgemäßes Verfahren durchzuführen. Weiterhin kann das Computerprogramm auf dem maschinenlesbaren Träger abrufbar gespeichert sein. Insbesondere kann es sich bei dem maschinenlesbaren Träger um eine CD, DVD, Blu-Ray Disc, einen Memory-Stick oder eine Festplatte handeln. Die Recheneinheit 7 kann in Form von Hard- oder in Form von Software ausgebildet sein. Beispielsweise ist die Recheneinheit 7 als ein sogenanntes FPGA (Akronym für das englischsprachige "Field Programmable Gate Array") ausgebildet oder umfasst eine arithmetische Logikeinheit.

In dem hier gezeigten Beispiel ist in einem Speicher 60 der Steuereinheit 7 wenigstens ein Computerprogramm gespeichert, welches alle Verfahrensschritte des erfindungsgemäßen Verfahrens durchführt, wenn das Computerprogramm auf dem Computer ausgeführt wird. Das Computerprogramm zur Ausführung der Verfahrensschritte des erfindungsgemäßen Verfahrens umfasst Programmcode. Weiterhin kann das Computerprogramm als ausführbare Datei ausgebildet sein und/oder auf einem anderen Rechensystem. Beispielsweise kann die medizinische Bildgebungsanlage so ausgebildet sein, dass die Recheneinheit 7 das Computerprogramm zum Ausführen des erfindungsgemäßen Verfahrens über ein Intranet oder über das Internet in seinen internen Arbeitsspeicher lädt.

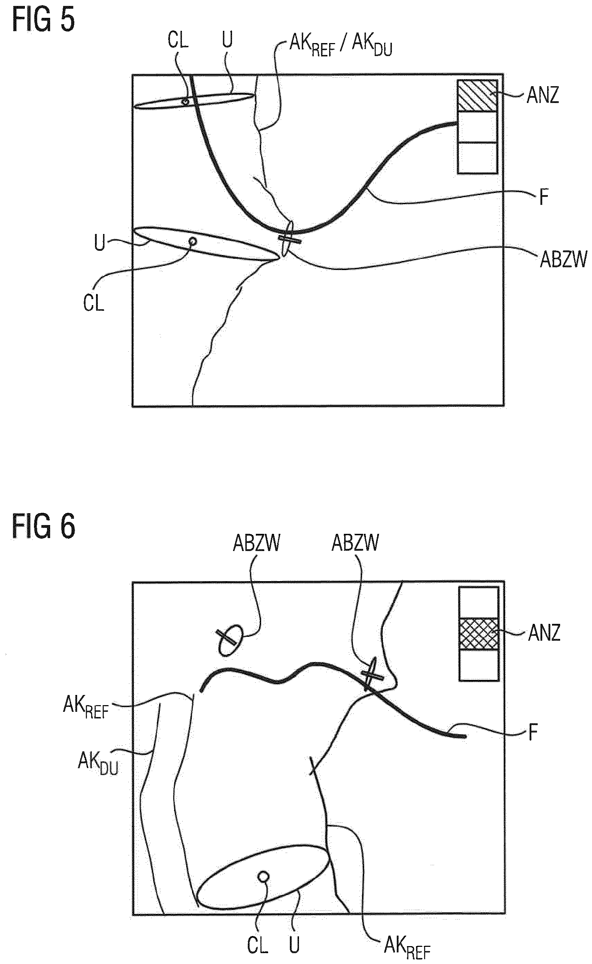

**Figur 5** und **Figur 6** zeigen jeweils ein Superpositionsbild SUP gemäß einem Ausführungsbeispiel der vorliegenden Erfindung. Betrachtetes Objekt ist auch hier ein Blutgefäß. Erkennbar sind jeweils segmentierte Centerlines CL eines Blutgefäßes, elliptisch approximierte Blutgefäßumfänge U, Außenkonturen AKREF des Blutgefäßes gemäß Referenzbild REF und Außenkonturen AKDU/AKMODU gemäß Durchleuchtungsbild DU/MODU, verschiedene Führungsdrähte F, elliptische approximierte Gefäßabgänge/Abzweigungen ABZW (sowie deren Centerlines) und vieles mehr. Insbesondere ist auch die Lage eines bereits eingebrachten Stentgrafts in dem Blutgefäß erkennbar (gestauchte Wellenlinien = Drahtgeflecht des Stentgrafts). In Figur 5 verläuft Führungsdraht F im Wesentlichen zentral durch die Gefäßabzweigung ABZW. Ferner verlaufen die Gefäßaußenkonturen AKREF und AKDU im Wesentlichen deckungsgleich. Insgesamt ist das Abweichungsmaß innerhalb eines ersten, akzeptablen Toleranzbereichs. Es erfolgt als Überlagerung zu dem Superpositionsbild eine Anzeige ANZ in Form einer Ampel, die hier auf 'grün' (oberes schraffiertes Kästchen) gestellt ist. Keine weiteren Korrekturschritte sind erforderlich. Bei dem in Figur 5 gezeigten Superpositionsbild kann es sich um ein ,einfaches', ein korrigiertes oder modifiziertes Superpositionsbild SUP, MODSUP, KORSUP handeln. In Figur 6 verläuft Führungsdraht F in deutlichem Versatz zur Centerline der Gefäßabzweigung ABZW. Ferner verlaufen die Gefäßaußenkonturen AKREF und AKDU in deutlichem Versatz zueinander. Insgesamt ist das Abweichungsmaß außerhalb des ersten, aber noch innerhalb eines zweiten, Toleranzbereichs. Mit anderen Worten ist die Überlagerung unter Anwendung einer Korrekturmethode noch nutzbar. Es erfolgt als Überlagerung zu dem Superpositionsbild eine Anzeige ANZ in Form einer Ampel, die hier auf 'gelb' (mittleres schraffiertes Kästchen) gestellt ist. Korrekturschritte sind erforderlich. Diese können wir oben beschrieben automatisch oder semi-automatisch durchgeführt werden.

In einen weiteren (nicht dargestellten) Fall können die ermittelten Abweichungen in dem Superpositionsbild außerhalb des zweiten Toleranzbereichs liegen. In diesem Fall erfolgt als Überlagerung zu dem Superpositionsbild eine Anzeige ANZ in Form einer Ampel, die hier auf 'rot' (unteres schraffiertes Kästchen) gestellt ist. Erfassung eines neuen, modifizierten Durchleuchtungsbildes, wie weiter oben beschrieben, kann angeraten sein.

Wo noch nicht explizit geschehen, jedoch sinnvoll und im Sinne der Erfindung, können einzelne Ausführungsbeispiele, einzelne ihrer Teilaspekte oder Merkmale mit einander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu.

## Patentansprüche

1. Verfahren zur automatischen Prüfung eines Superpositionsbildes (SUP, MODSUP, KORSUP) einer interessierenden Körperregion eines Untersuchungsobjektes (6), umfassend folgende Schritte:
- Ermitteln (S11, S21, S31) wenigstens einer Referenzposition eines Objektes (AKREF, U, CL, ABZW) in einem Referenzbild (REF),
- Ermitteln (S12, S221, S222, S32) einer aktuellen Position des Objektes (AKDU) in einem aktuellen Durchleuchtungsbild (DU, MODU),
- Erzeugen (S13, S231, S232, S33) des Superpositionsbildes durch Überlagern des aktuellen Durchleuchtungsbildes und des Referenzbildes,
- Ermitteln (S14, S241, S242, S341, S342) wenigstens eines Parameters (PAR ABW, PAR KORABW, PAR MODABW) kennzeichnend ein Abweichungsmaß (ABW, KORABW, MODABW) zwischen Referenzposition und aktuelle Position des Objektes in dem Superpositionsbild,
- Anzeigen (S15, S251, S252, S351, S352) des Abweichungsmaßes für einen Benutzer.

2. Verfahren nach Anspruch 1, wobei die Schritte
- Ermitteln einer aktuellen Position des Objektes in dem aktuellen Durchleuchtungsbild,
- Erzeugen des Superpositionsbildes durch Überlagern des aktuellen Durchleuchtungsbildes und des Referenzbildes,
- Ermitteln wenigstens eines Parameters kennzeichnend ein Abweichungsmaß zwischen Referenzposition und aktueller Position des Objektes in dem Superpositionsbild, und
- Anzeigen des Abweichungsmaßes für einen Benutzer in Quasi-Echtzeit ausgeführt werden.

3. Verfahren nach einem der Ansprüche 1 oder 2, wobei das Ermitteln der aktuellen Position des Objektes ein Segmentieren eines in dem Blutgefäß befindlichen Instruments, einer Endoprothese, der Außenkontur und/oder des Volumens des Objektes umfasst.

4. Verfahren nach Anspruch 3, wobei die Segmentierung unter Anwendung eines neuronalen Netzes oder mittels einer Template-Matching Technik erfolgt.

5. Verfahren nach einem der vorherigen Ansprüche, wobei die Schritte
- Ermitteln einer aktuellen Position des Objektes in dem aktuellen Durchleuchtungsbild,
- Erzeugen des Superpositionsbildes durch Überlagern des aktuellen Durchleuchtungsbildes und des Referenzbildes,
- Ermitteln wenigstens eines Parameters kennzeichnend ein Abweichungsmaß zwischen Referenzposition und aktueller Position des Objektes in dem Superpositionsbild, und
- Anzeigen für einen Benutzer des Abweichungsmaßes kontinuierlich (i) für eine Vielzahl aufeinander folgender Durchleuchtungsbilder (DUi) durchgeführt werden.

6. Verfahren nach Anspruch 5, wobei das Ermitteln des Parameters kennzeichnend das Abweichungsmaß Vorwissen über segmentierte Strukturen aus wenigstens einem zuvor analysierten Durchleuchtungsbild (DUi-1) berücksichtigt.

7. Verfahren nach einen der vorherigen Ansprüche, welches auch umfasst
- Basierend auf dem Parameter kennzeichnend das Abweichungsmaß Ableiten (S26) wenigstens eines Aufnahmeparameters (PAR ACQU) für eine medizinische Bildgebungsanlage (1), wenn der Parameter für das Abweichungsmaß einen vorbestimmten Schwellwert (S2) überschreitet
- Erfassen (S27) eines modifizierten Durchleuchtungsbildes (MODU) unter Anwendung des Aufnahmeparameters
- Ermitteln (S222) einer aktuellen Position des Objektes in dem modifizierten Durchleuchtungsbild
- Erzeugen (S231) eines modifizierten Superpositionsbildes (MODU) durch Überlagern des modifizierten Durchleuchtungsbildes und des Referenzbildes
- Ermitteln (S242) wenigstens eines Parameters kennzeichnend ein modifiziertes Abweichungsmaß (PAR MODABW) zwischen Referenzposition und aktueller Position des Objektes in dem Superpositionsbild
- Anzeigen (S252) des modifizierten Abweichungsmaßes (MODABW) für einen Benutzer.

8. Verfahren nach einem der vorherigen Ansprüche, welches auch umfasst
- Automatisches Erzeugen (S36) eines korrigierten Superpositionsbildes (KORSUP) basierend auf dem Parameter kennzeichnend das Abweichungsmaß zwischen Referenzposition und aktueller Position des Objektes
- Ermitteln (S342) wenigstens eines Parameters kennzeichnend ein korrigiertes Abweichungsmaß (PAR KORABW) zwischen Referenzposition und aktueller Position des Objektes in dem korrigierten Superpositionsbild und
- Anzeigen (S352) des korrigierten Abweichungsmaßes (KOR ABW) für einen Benutzer.

9. Recheneinheit (7) zur automatischen Prüfung eines Superpositionsbildes (SUP, KORSUP, MODSUP) einer interessierenden Körperregion eines Untersuchungsobjektes (6), aufweisend Mittel (60, 8) zum Durchführen eines Verfahrens nach einem der Ansprüche 1 bis 8.

10. Recheneinheit nach Anspruch 9, die mit einer medizinischen Bildgebungsanlage (1) derart verbunden ist, dass Steuersignale in Bezug auf korrigierte Aufnahmeparameter (PAR ACQU) bzw. aktuelle und/oder modifizierte Durchleuchtungsbilder (DU/MODU) übertragen werden können.

11. Medizinische Bildgebungsanlage (1) in Form eines C-Bogen-Röntgengerätes, umfassend eine Recheneinheit (7) gemäß einem der Ansprüche 9 oder 10.

12. Computerprogramm mit Programmcode, um das Verfahren nach einem der Ansprüche 1 bis 8 durchzuführen, wenn das Computerprogramm auf einem Computer ausgeführt wird.

13. Computerlesbarer Datenträger mit Programmcode eines Computerprogramms, um das Verfahren nach einem der Ansprüche 1 bis 8 durchzuführen, wenn das Computerprogramm auf einem Computer ausgeführt wird.
